# EUROPEAN PATENT APPLICATION

(11) **EP 2 039 292 A1**
(43) Date of publication of application: **25.03.2009**
(21) Application number: 07794066.6
(22) Date of filing: 25.06.2007
(51) Int. Cl.: A61B 5/117, G06K 9/00

(54) **FINGERPRINT RECORDING DEVICE**

(30) Priority: 10.07.2006 RU 2006124769
(71) Applicant: Zaitsev, Pavel Anatolievich, Chelyabinskaya obl. 456-318 (RU); Bichigov, Vladimir Nikolaevich, Chelyabinskaya 456-320 (RU); Moksin, Alexandr Vladimirovich, Chelyabinskaya obl. 456-318 (RU); Chernovol, Alexei Nikolaevich, Chelyabinskaya obl. 456-320 (RU); Shapshal, Ivan Borisovich, Chelyabinskaya obl. 456-318 (RU)
(72) Inventor: Zaitsev, Pavel Anatolievich, Chelyabinskaya obl. 456-318 (RU); Bichigov, Vladimir Nikolaevich, Chelyabinskaya 456-320 (RU); Moksin, Alexandr Vladimirovich, Chelyabinskaya obl. 456-318 (RU); Chernovol, Alexei Nikolaevich, Chelyabinskaya obl. 456-320 (RU); Shapshal, Ivan Borisovich, Chelyabinskaya obl. 456-318 (RU)
(74) Representative: Sloboshanin, Sergej
(86) International application number: PCT/RU2007/000345
(87) International publication number: WO 2008/008000

(57) **Abstract**

The invention relates to optically forming fingerprint pictures, in particular to device for producing fingerprint cards used by low-enforcement authorities. The aim of said invention is to improve the quality of fingerprint recording by removing a moisture influence on a obtainable picture without the low-size of the device. The inventive fingerprint recording device comprises a prism having at least three working faces, wherein the first face is adjacent to the second and third faces, optically interconnected radiation sources, a projection system and a system of photoreceiving elements, which are positioned in front of the second prism face, and a signal processing unit which is electrically connected to the system of photoreceiving elements, wherein the first face is used for perceiving fingerprints, the second face is simultaneously used as the input and output face for a light beam, the third face of the prism is embodied such that it is reflecting and is provided with a mirror coating, the angle between the first and second faces ranges from 90 to 62°, the angle between the first and third faces ranges form 76 to 90° and the second prism face is used as the input radiation refracting face.

## Description

The utility model relates to the field of the optical formation of images of fingerprints, particularly to apparatus for obtaining fingerprint cards used by judicial authorities.

A technical solution according to US Patent 5625448 is known, comprising a prismatic means consisting of three separate prisms abutting each other by corresponding sides. Said apparatus is characterized in that the prismatic means is hard to manufacture since must be arranged very accurately.

The technical solutions of RU 2185096 and RU 2218866 are known, comprising a prism and - optically connected to one another - a light ray source, a projection system, a system of photoreceiving elements, a signal processing unit, which is electrically connected to the system of photoreceiving elements. One of the sides of the prism is the receiving surface for the prints of the fingers (the first side); the one adjacent to the first side (the second side) is the entry side (provided for the inlet of an entering beam of rays into the prism for illuminating the receiving side), and at the same time the exit side (provided for the outlet of an exiting beam for the formation of an image of the receiving side in the photoreceiving system); the third side of the prism is the reflection side.

The prism of the apparatus of RU 2185096 comprises four working sides and the prism of the apparatus of RU 2218866 comprises three working sides.

In the mentioned solutions the sizes of the angles of the adjacent sides of the prism (to the first side) are chosen based on the condition for obtaining the best image of the fingerprint, to this end the sighting angle of the receiving (first) side is chosen equal to the illuminating angle and larger than the angle of total internal reflection on the boundary between prism and air.

However, drops of sweat and moisture on the fingers have an adverse influence on the quality of the image of the prints, since the sighting angle of the receiving (first) side is smaller than the angle of total internal reflection on the boundary dividing glass and water. Thus the rays illuminating the receiving (first) side on the portion where the drop of moisture contacts the prism exit the prism into a drop and are scattered, not reflected. For this reason the drops of water or sweat can cause stains on the image which show little difference to traces of skin contact, which distorts the obtained image of the fingerprint.

The position of the light source and the projection system on one side of the prism results in a reduction of the dimensions of said apparatus and characterizes the compact build of the design layouts.

The apparatus of RU 2218866 is chosen as the most pertinent prior art.

The object of the claimed technical solution lies in the enhancement of the quality of the fingerprint recording by means of preventing an influence of moisture on the obtained image while maintaining the compact build of the apparatus.

Said object is achieved in that the apparatus for fingerprint recording comprises a prism with at least three working sides, the first side being adjacent to the second and third sides; a source of light rays, a projection system and a system of photoreceiving elements which are optically connected to each other and situated opposite the second side of the prism, a signal processing unit which is electrically connected to the system of photoreceiving elements, the first side being the receiving side for the fingerprints, the second side being the entry and simultaneously the exit side for the light ray beam, the third side of the prism being the reflecting side and having a mirror coating, the angle between the first and the second side being 90-62 degrees, the angle between the first and the third side being 76-90 degrees, the second side of the prism being the refractive side for the entering light rays.

For preventing an influence of moisture, the geometric angles between the adjacent sides of the prism - the working sides - are chosen based on the condition that the angle of incidence and the angle of reflection on the receiving side of the prism have a value which is lower than the angle of total internal reflection on the glass-skin boundary but larger than the angle of total internal reflection on the glass-water boundary.

It is known that the angle of total internal reflection on the glass-water medium division boundary equals 61.7 degrees, while the angle of total internal reflection on the glass-skin medium division boundary equals 71.3 degrees for optical glass with a refractive index of 1.52.

Obtaining the best image of a fingerprint becomes possible due to the range of values of angles of internal reflection from the first receiving side, constituting 61.7-71.3 degrees, while the beam of light rays on the boundary of contact of a drop of moisture with the prism will always be reflected from the receiving side.

This range of values predetermines the calculation of values of the geometric angles of the prism.

To guide the beam reflected from the receiving side out of the prism through the second, exit side, it is reflected from the third side in such a way that it is directed parallel to the first, receiving side, or at a small angle to that side, in a direction away from it. For the beam reflected from the receiving side at an angle exceeding 61.7 degrees, the angle between the first and the third side must exceed 75.8 degrees. If this angle is 90 degrees, the exiting beam within the prism will have a direction parallel to the direction of the entering beam between the working sides of the prism. This condition means that the compact build of the apparatus can be maintained.

If the angle between the first and the third side is larger than 90 degrees, the exiting beam would bend from the direction of the entering beam sideways away from the receiving side. This would render it necessary to shift the projection system sideways from the receiving side as well, which would result in an increase of the dimensions of the apparatus.

Thus, in the claimed apparatus the angle between the first receiving side and the third reflecting side is 76-90 degrees.

With such a range of values of the angles between the sides, the light beam reflected from the first side at an angle of 62 degrees (approximately 61.7 degrees) falls on the third side at an angle of 14-28 degrees. The direction of the beam reflected from the third side is 0-28 degrees with respect to the first side.

The beam reflected from the first side at an angle of 70 degrees falls on the third side at an angle of 10-20 degrees. The direction of the beam reflected from the third side is 0-20 degrees with respect to the first side.

The best conditions for guiding the exiting beam out of the prism are reached in a condition when the second side of the prism is perpendicular to the exiting beam; for the angle between the first and the third side of 76-90 degrees, the second side which is perpendicular to the beam of light rays is 90-62 degrees, correspondingly.

Due to the range of values of the geometrical angles of the prism obtained by calculation on the basis of the angle of total internal reflection of the light rays from the receiving side, it becomes possible to obtain the technical result.

For a fuller reflection of the light beam from the third side, the latter is provided with a mirror coating.

Thus, the entire range of values of the two adjacent geometrical angles of the prism creates the possibility of reflecting the light beam from the receiving side on the glass-water medium division boundary, preventing an influence of moisture drops on the quality of the image of the fingerprint.

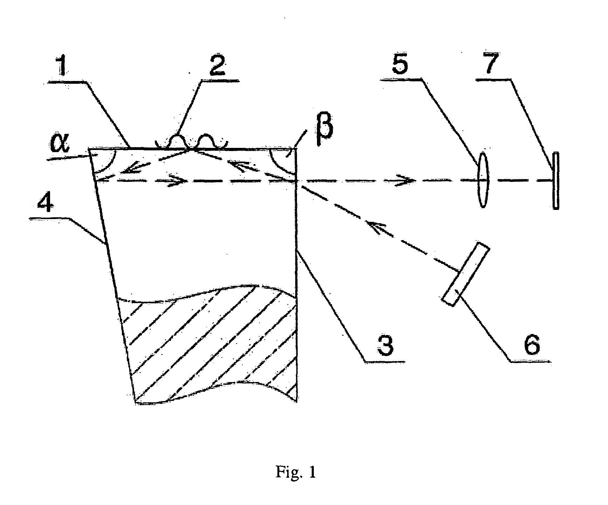

The claimed utility model is explained by the drawing (see Fig. 1).

The apparatus for fingerprint recording comprises a prism with three working sides, the first side 1 of which is the one that receives the fingerprints 2, the second side 3 is the entry and exit side for the light beam, the third side 4 is the one that reflects the light beam. The apparatus also comprises an optical projection system 5, a light source 6, and a system of photoreceiving elements 7. The elements 5, 6, 7 of the apparatus are arranged opposite the side 3 of the prism. The adjacent angle α between sides 1 and 4 is 76-90 degrees, the adjacent angle β between sides 1 and 3 is 90-62 degrees.

The axis of the light beam in the drawing is illustrated by a dashed line with arrows.

The surface of the sides of the prism near the angle situated between sides 3 and 4 forms a dead zone in the section of the prism, since no light beam enters or exits it. To reduce the dimensions of the prism, it can be cut off at random. The cut-off part is hatched in the drawing.

To describe the function of the claimed apparatus, the values of the geometrical angles are as follows: the angle α is 78 degrees, the angle β is 90 degrees.

The apparatus works as follows.

The light beam from the source 6, after being refracted, enters the prism (through the side 3) and falls on the side 1 (and is reflected from the side 1) at an angle of 66 degrees, undergoing total internal reflection. From the side 4 the light beam is reflected at an angle of 12 degrees to the side 4, runs parallel to the side 1 and exits the prism through the side 3 perpendicular to the side 3.

The formation of the fingerprint is carried out as follows. When no finger 2 is pressed on the prism, the side 1 fully reflects the light beam incident on it, while when the surface 1 is touched by a finger in places where the ridges of the fingerprint of the skin are pressed to the surface, part of the light rays leaves the prism; the total internal reflection is destroyed, and light exits onto the skin. The reflected light rays exit the prism and fall onto the projection system 5, forming an image of the touching finger on the system of photoreceiving elements 7.

When moisture is present on the finger, the light beam undergoes total internal reflection and does not exit the prism into the drop. Thus the system of photoreceiving elements generates an adequate image of the fingerprint without distortions.

## Claims

1. pparatus for fingerprint recording, comprising a prism with at least three working sides, the first side being adjacent to the second and third sides; a source of light rays, a projection system and a system of photoreceiving elements which are optically connected to each other and situated opposite the second side of the prism, a signal processing unit which is electrically connected to the system of photoreceiving elements, the first side being the receiving side for the fingerprints, the second side being the entry and simultaneously the exit side for the light beam, the third side of the prism being the reflecting side and having a mirror coating, the angle between the first and the second sides being 90-62 degrees, the angle between the first and the third sides being 76-90 degrees, the second side of the prism being the refractive side for the entering light rays.
